# EUROPEAN PATENT APPLICATION

(11) **EP 1 559 364 A1**
(43) Date of publication of application: **03.08.2005**
(21) Application number: 05075461.3
(22) Date of filing: 20.08.2002
(51) Int. Cl.: A61B 5/00

(54) **Wireless diabetes management devices and methods for using the same**

(30) Priority: 20.08.2001 US 313833 P
(62) Divisional of application: 02755215.7
(71) Applicant: Inverness Medical Limited, Inverness IV2 3ED (GB)
(72) Inventor: Moerman, Piet, 9831 St. Martens-Latem (BE); Scott, David, Witney Oxfordshire, OX28 3SZ (GB); Mcaleer, Jerry, Wantage Oxfordshire, OX12 0NR (GB)
(74) Representative: Mercer, Christopher Paul

(57) **Abstract**

Advanced disease state management for diabetics through a wireless phone. A remote counselling service is accessible through the wireless communication feature of a wireless phone that interfaces to the diabetics' glucometer. A patient can send his glucose data directly to a counselling centre and receive a message back on how to proceed with treatment. Wireless technology can additionally be utilised to alert a caretaker that a hypoglycaemic value has been measured. The caretaker can then intervene to secure help. The telemetry can also be used to measure compliance with a prescribed disease management regime. The disease management system is also suitable for targeted marketing of supplies to diabetic patients.

## Description

### Field of the Invention

The present invention relates to devices for remote disease state management and, in particular, for the management of diabetes using wireless reporting devices in conjunction with an automated monitoring centre.

### Background of the Invention

Diabetes mellitus is a serious, life-long disorder that is, as yet, incurable. Each year, between about 500,000 and about 700,000 people are diagnosed with diabetes, one of the leading causes of death and disability in the United States. In 1993, there were approximately eight million diagnosed cases of diabetes mellitus in the United States and the number has most likely grown to about 10 million currently diagnosed cases. This number represents only those people *diagnosed* with diabetes mellitus, estimated to be less than half the number of actual diabetics in the United States.

The effects from diabetes on the health care system are startling. In the U.S., the cost of hospitalisations, supplies, lost work, disability payments and premature deaths from diabetes reached more than $92 billion in 1992 alone. In addition, the long-term complications associated with diabetes, particularly when poorly managed, can lead to serious financial and human consequences. Serious diabetes-related complications, including cardiovascular disease, kidney disease, nerve damage, blindness, circulatory problems (which can lead to amputations), stroke, heart disease and pregnancy complications, are estimated to cost more than $24 billion annually. Some health maintenance organisations estimate that while only 3.1% of their covered patients have diabetes, diabetic patients account for over 15% of their total healthcare costs.

Research, including the Diabetes Control and Complications Trial (DCCT) conducted by the National Institute of Health in 1993, has shown that if people with diabetes closely monitor and control their blood glucose levels, they will enjoy significant health benefits. Consistent management of diabetes, which includes diet, exercise and aggressive monitoring and control of blood glucose levels, can lessen the risk of serious complications and potentially reduce some diabetes-related conditions by more than half.

The DCCT study was the largest, most comprehensive diabetes study ever conducted, involving 1,441 volunteers with Type I diabetes and managed through 29 medical centres across the United States and Canada. The study compared the effects and results of two treatment regimens: standard therapy and intensive control. According to the standard therapy regimen, a diabetic follows a fixed insulin schedule independent of glucose levels or dietary intake. The intensive control regimen requires diabetics to actively monitor glucose levels and other disease-impacting factors and make insulin-dosing decisions based on actual glucose test results and dietary intakes. The DCCT study revealed that active management of diabetes according to the intensive control regimen could, among other benefits, reduce eye disease by up to 76%, reduce kidney disease by up to 50% and reduce nerve disease by up to 60%. Strategies for diabetes management recommended by DCCT researchers include the development and implementation of individualised care management programs, with emphasis on in-depth patient education.

The intensive control treatment regimen mimics the functionality of a normal, healthy pancreas more closely, and provides significant medical benefits to diabetics who follow the intensive control treatment regimen. Consequently, since the DCCT trial results, a large number of the diabetes community in the United States has switched to the intensive control treatment regimen. However, for the majority of the diabetics, this new regimen is a real challenge and is difficult to follow. Rather than following a straightforward and fixed insulin schedule prescribed by a health care professional, the intensive control treatment regimen requires complex decisions on the part of the diabetic patient in response to a number of variables, including stress, exercise, food intake, insulin, adrenaline and other hormones. A diabetic patient must personally decide on an appropriate treatment in real-time and in response to changing variables. Under the intensive control treatment regimen, a diabetic individual is required to test blood glucose levels frequently. However, the pain, discomfort, cost and time involved often deter patients from performing frequent testing. In addition, many health plan providers do not have the time, resources, funds or expertise to implement appropriate disease management programs to assist diabetic patients. There is a distinctive need for assisting a diabetic in making therapeutic decisions and encouraging frequent testing and monitoring of blood glucose levels. Without assistance in the decision making process, the new intensive control treatment regimen may fail to deliver its potential benefits.

Furthermore, the new intensive control treatment regimen necessitates more tightly controlled glucose levels, which inherently cause an increased risk of more frequent hypoglycaemic episodes. A very real issue facing many diabetics is the fear and possibility of falling into a hypoglycaemic coma, or experiencing other diabetic emergencies, without external assistance. Likewise, the fear of a diabetic emergency in a child or other dependent confronts many parents and guardians of diabetic individuals. The possibility of a diabetic emergency hinders both the diabetic individual and the guardian from leading active, independent lifestyles. A system which would assist with these challenges would be invaluable to the motivated diabetic audience.

### Summary of the Invention

The present invention provides a system for monitoring and assisting in the treatment of a patient, independent of the location of the patient, comprising: a monitoring centre; a meter for measuring the level of an analyte in said patient; and a communications device adapted for communication with said monitoring centre and said meter, wherein said communications device is adapted to transmit information related to said analyte level in said patient to said monitoring centre.

In a preferred embodiment, the patient is a diabetic patient and the meter is a glucose meter. The communications device can be either a wireless communications device or a communications device that transmits information via the internet.

In a preferred embodiment the wireless communications device can access a remote counselling centre. A user can send his glucose data directly to the counselling centre and receive feedback from the centre regarding an appropriate treatment in response to the glucose data. The wireless communication feature may also be utilised to communicate with and/or alert a caretaker (parent of a diabetic child, the school nurse or the neighbour of an elderly diabetic) that a hypoglycaemic value has been recorded on the dependent's monitor. The caretaker can then intervene to secure help. The telemetry of the present invention is also suitable for measuring compliance with a prescribed disease management regime. For example, the caretaker can check whether a child performed a required glucose test and the actual results of the glucose test.

The communications device of the present invention provides constant and convenient assistance independent of the location of the user. In addition to assisting in making complex decisions and clarifying glucose readings, the present invention encourages diabetic patients to adhere to an intensive treatment plan and significantly improves the health of a user. The present invention further provides a safeguard against diabetic emergencies.

The present invention may, in particular, provide assistance to a diabetic individual through a wireless phone or other hand-held communications device. The disease state management services offered by the present invention may include, in various embodiments, diabetes counselling, emergency services, diabetes supervision, supply ordering, diabetes news, diabetes watch for health care providers and patient location information via GPS.

The present invention will be discussed below in connection with a wireless telephone, although those of ordinary skill will recognise that other types of personal communications devices can also be used.

For example, the user may access the management services offered by the present invention using any one of the following devices: a wireless application protocol (WAP) compliant mobile phone, a wireless connected personal digital assistant (PDA) or a laptop or desktop PC. Any compatible device must have a port (e.g. RS232, IR, USB, bluetooth or other external port) for data connectivity connection to a glucose meter.

A compatible WAP compliant mobile phone would need to have a minibrowser (for example UP version 1.2 or greater). The preferred phone may be configured to operate on a mobile network using Code Division Multiple Access (CDMA). Alternatively, the phone may operate on a network using GSM. A compatible PC would need either wireless ability or internet access. Wireless data transfer is preferred although data could be transferred to the database via a PC.

Any of the devices mentioned above can be used in conjunction with any data port or interface capable glucose meter. Alternatively, a proprietary "clip-on" glucose meter may be used in conjunction with a WAP compliant mobile or a wireless PDA. Any compatible mobile phone or PDA may utilise a WAP Gateway to access the internet. This would allow the applications (i.e. glucose interpretation, e-mail, paging and other applications) to be device independent. The system may also allow for "push" capability, meaning that an e-mail could be sent which has an active URL embedded in the message. A compatible laptop or desktop PC may access the internet by its normal dial-up method. To assure security of data and commercial information, the mobile phone or PDA may utilise Wireless Transport Layer Security (WTLS) whilst the laptop or desktop may utilise Transport Layer Security (TLS) (also known as Secure Socket Layer (SSL)).

### Brief Description of the Drawings

Figure 1 is a diagram illustrating a wireless diabetes management system according to one embodiment of the present invention.
Figure 2 is a diagram illustrating the data flow among components of a wireless diabetes management system according to another embodiment.

The foregoing and other objects, features and advantages of the invention will be apparent from the following description and from the accompanying drawings.

### Detailed Description of the Invention

The present invention will be described below relative to an illustrative embodiment. Those skilled in the art will appreciate that the present invention may be implemented in a number of different applications and embodiments and is not specifically limited in its application to the particular embodiment depicted herein.

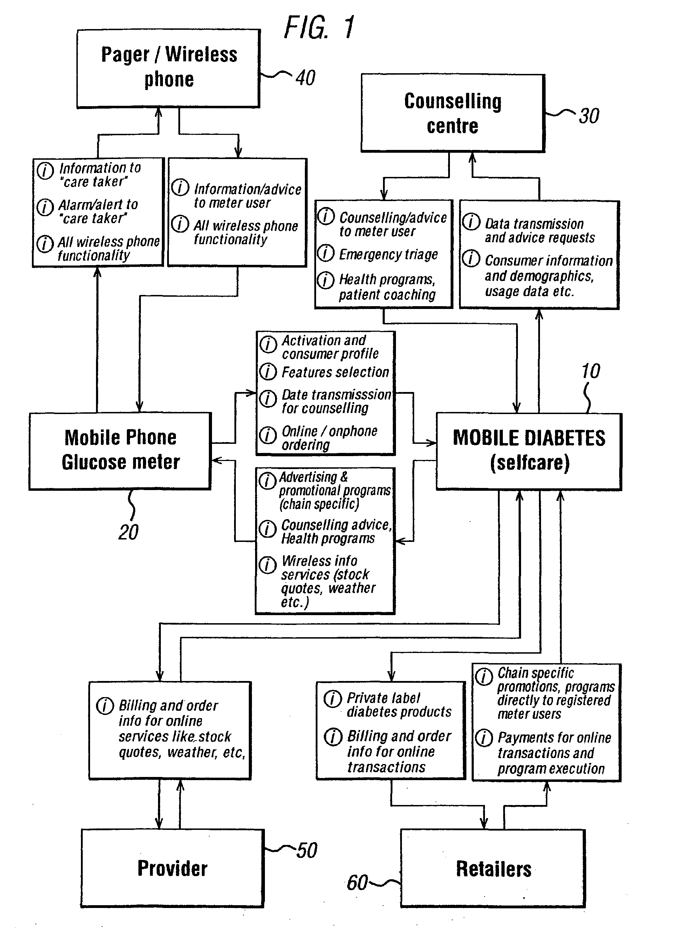

Figure 1 depicts a wireless diabetes management system to provide assistance to a diabetic patient independent of the location of the diabetic patient. The diabetes management system of the illustrative embodiment includes an automated diabetes monitoring centre (10) that receives data from a remote glucose meter operating in conjunction with a mobile wireless telephone (20). According to one aspect of the present invention, the glucose meter connects to and transfers data to a patient's wireless phone (20), which then transfers blood glucose measurements and/or other diabetes-related information to the diabetes monitoring centre (10). According to another aspect, the glucose measurements and other diabetes-related information can also be passed to the phone or pager of a caretaker (40), who can then provide assistance or advice to the diabetic patient. In addition to providing diabetes assistance, the wireless phone (20) may be utilised for standard voice communication between the caretaker and the user. Data received from the glucose meter via the wireless phone (20) is processed by the automated monitoring centre (10) and may be passed as needed to a counselling centre (30), a provider (50) and/or a retailer (60). In response, the counselling centre (30), the provider (50) and/or the retailer (60) can return data to the automated monitoring centre (10) which can then be passed to the patient via the remote wireless phone (20). If the patient requests advice regarding a blood glucose measurement, the counselling centre (30) performs an analysis of the data provided by the meter through the phone (20) and provides an appropriate treatment response to the meter user. If the data reveals a life-threatening blood glucose level or other serious condition, the counselling centre (30) may contact emergency services to aid the patient. The monitoring centre (10) may further provide health programs and patient coaching to the diabetic patient to assist in the management of diabetes. The provider (50) may provide on-phone information, such as e-mail, weather and stock quotes, to the patient by means of the wireless phone (20). The retailer (60) may provide diabetes products, information and advertisements to the diabetic patient via the wireless phone (20).

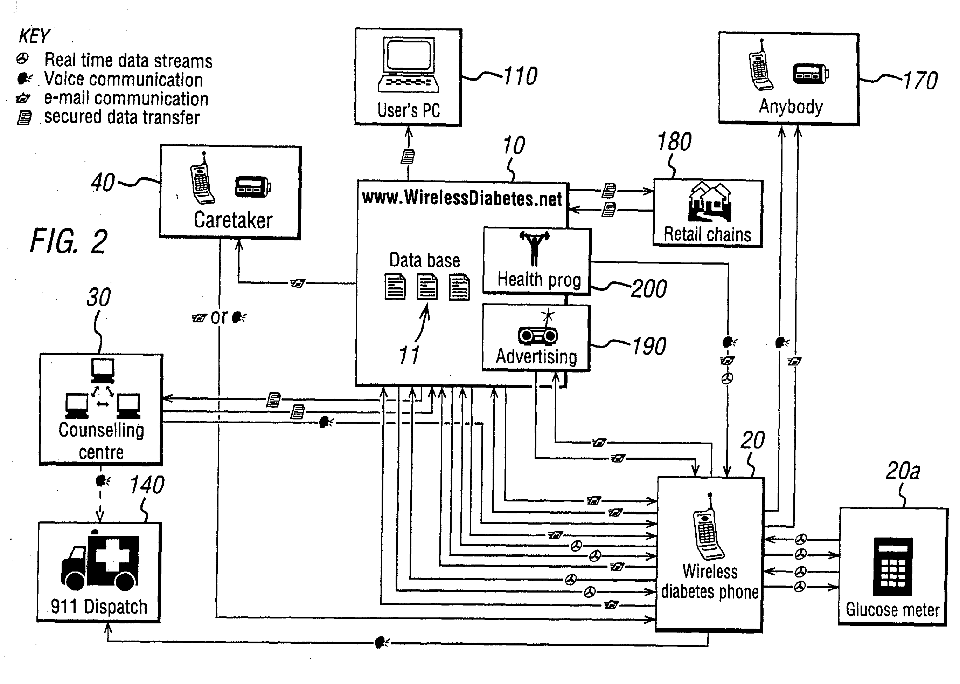

Figure 2 illustrates the flow of data between the various components of the diabetes management system according to the illustrative embodiment of the invention. In the depicted embodiment, the automated monitoring centre (10) comprises a database (11), which receives data from and sends information to the wireless diabetes phone (20). The wireless diabetes phone (20) transmits and receives information from a glucose meter (20a) which measures and records glucose levels in the patient. The wireless diabetes phone also operates as a regular wireless telephone and may include Web Application Protocol (WAP) capabilities to make the phone compatible with certain "text" web sites using a WAP browser. The wireless diabetes phone may include a feature to automatically contact emergency services, illustrated as an ambulance (140), in an emergency situation, such as a life-threatening glucose level in the patient. The automated monitoring centre (10) may pass data as needed between the wireless diabetes phone (20) and the counselling centre (30), the caretaker (40) or an additional party (170). The additional party (170) may be a health care provider, a managed care system or any person with whom the user wishes to communicate. The automated monitoring centre (10) may further pass data as needed between the wireless diabetes phone (20) and a retail store (180), an advertiser (190) or a health program (200) included in the automated monitoring centre (10). The automated monitoring centre (10) may also be accessed over a data network, such as the Internet, by a personal computer (110).

According to the illustrative embodiment, the wireless phone (20) utilises real-time data streams to interact with the glucose meter (20a). The wireless diabetes phone (20) may communicate with the caretaker (40), additional party (170), the monitoring centre database (11) and the counselling centre (30) through voice communication and/or e-mail communication. The wireless diabetes phone (20) may directly contact an emergency service (140) via voice communication in case of an urgent situation. The counselling centre (30) may also contact emergency services via voice communication, if necessary. The health program (200) may provide coaching and other instructions to the user via voice or e-mail communication. Advertising may be sent to the wireless diabetes phone (20) via the automated monitoring centre (10) using e-mail communication. Secured data transfer may be utilised to transmit information between the monitoring centre database (11) and the counselling centre (30), the retail store (180) and the personal computer (110) of the diabetic patient or other party.

The features and components of the diabetes management system will be described in detail below.

### The Wireless Diabetes Phone

The diabetes management device and system of the illustrative embodiment of the present invention comprises a glucose meter (20a) which acquires wireless communication by being attached to a digital WAP-enabled wireless phone (20). According to the illustrative embodiment, the wireless diabetes phone (20) is provided with a connector cable to ensure easy connection and disconnection with the glucose meter (20a). According to alternate embodiments, the glucose meter comprises a temporary or permanent attachment to a data port on the phone. The glucose meter (20a) may comprise a clip-on module configured to attach directly to the wireless diabetes phone (20), or may be internally built into the phone housing. The clip-on module may comprise a strip connector and an Application Specific Integrated Circuit (ASIC) for signal processing. Alternatively, the phone may be configured to attach directly to any glucose meter. The glucose meter (20a) may include data collection, storage and displaying capabilities to measure and record diabetes-related information, such as glucose data, measured insulin dosages, exercise, dietary intake, blood pressure and heart rate readings. The wireless diabetes phone accepts the glucose, diet and insulin data stored in the glucose meter and transmits the data to the monitoring centre database (11). The wireless diabetes phone may gather additional information not provided by the glucose meter through direct input into the phone using the phone's display and keypad. For example, the user can specify, during the activation process, which meter he is using. The wireless diabetes phone may then receive the appropriate interpretation protocol for the user's meter. The display may show information, such as the time, date, glucose test result, historical results, graphical representations of historical values, and so on. The wireless diabetes phone menus may also be customised to show features selected by the user.

The wireless communication of the present invention has the additional benefit of being bi-directional. Messages sent using the wireless diabetes phone (20) can have different functions, including standard voice communication. The system may not be restricted to communicating strictly disease-related issues. The combined phone-meter can be used as a standard wireless phone, as well as a receiver and sender of web information (like weather, stock quotes and e-mail).

### Emergency Services

The wireless diabetes phone (20) may include an alarm feature that is triggered if a dangerous blood glucose level is measured by the associated glucose meter (20a). If a glucose reading indicates that the user is in need of emergency assistance, a pre-dial 911 "Emergency Care" function on the wireless diabetes phone (20a) of the present invention can be used to summon emergency services (140). When a measured glucose level exceeds a prescribed range, the wireless diabetes phone triggers an alarm and automatically summons help for the diabetic patient. Additionally, a neighbour, parent or other caretaker (40) can be alerted that the user needs help when the "Emergency Care" function is selected. These services can be used for calling help where assistance from the 911 services is not required. The caretaker can then call the patient or start organising help to intervene. The caretaker should have a device capable of receiving voice or text files, so that the alert can reach the caretaker as a pager or phone message. For example, an autodial voice message can be sent to the caretaker's phone, informing the caretaker that the user needs help. A pre-set text message can also be sent at the same time. The phone or pager number of the caretaker as well as the pre-set text file for the message may have been entered during activation of the phone through the website. In addition, the caretaker may access the central database to review the dependent's last glucose test results to provide further assistance in an emergency situation. The user may select an emergency care option in which their glucose data are sent to the data base. The data base can then send the latest glucose data and any other information to the caretaker as a text file. The caretaker would then be able to contact his dependent and/or log on to his dependent's website to review the data. The counselling centre (30) may also summon emergency services if the counsellor determines that the patient is in danger.

### Diabetes Professional Watch

The wireless diabetes phone (20) of the illustrative embodiment of the present invention provides a monitoring service offered by the counselling centre (30) or the person's health care provider. Each time a dangerous trend or value is generated on the glucose meter (20a), the wireless diabetes phone (20) of the present invention raises a warning and automatically transmits a message, detailing the latest glucose, insulin and diet data, to the counselling centre (30) or health care professional. The monitoring service allows the counselling centre or the health care professional to intervene when necessary. The wireless diabetes phone or glucose meter may be programmed to automatically recognise the dangerous trend or value. Patient measurements may be compared to programmed parameters in the telephone or glucometer to determine when a dangerous condition exists.

### Patient Location via GPS

According to yet another aspect, the wireless diabetes phone may further incorporate a GPS (global positioning satellite) system to allow the 911 services or other third parties to precisely locate the diabetic individual in case of an emergency. The GPS system provides an additional safeguard for a diabetic who may become disoriented or lost, for example if the diabetic has become hypoglycaemic.

### Central Diabetes Monitoring Centre

The monitoring centre database (11) controls the disease related information flow to and from the wireless diabetes phone (20). The database, part of the remote diabetes monitoring centre (10), registers users of the disease state management device and system of the present invention and records, manages and organises a variety of individual patient information (for example: glucose readings, reports and patient profiles, order details and credit card information). The database can also be used to distribute wireless diabetes phones to diabetics. The database serves to route messages between diabetics and third parties that provide the described diabetic services (remote counselling, on-line shopping, retailing, etc). According to the illustrative embodiment, the database includes a web site, which performs a variety of functions. The web site initially allows diabetic patients to register and activate the wireless diabetes phone (20). The web site can also be used to promote the diabetes services and to collect important information for establishing the communication between the user's glucose meter and his/her mobile phone. Once a user has been registered, the web site records and stores information about the user, such as glucose and therapy data, personal data, information regarding the patient's insurance plan, address, treatment regime, credit card details, health care professional details and so on. The site also allows users to access and change their information once it has been entered. A registered user of the web site, such as the diabetic patient, a caretaker, a managed care provider or a health care professional, can log on to the web site and access the services and information provided. For example, a health care professional can log on to access patient data, monitor a patient's diabetes management and formulate advice for the patient. This advice can then be sent directly to the wireless phone of the patient.

Such a website may reside on a dedicated server, behind a host-managed firewall. The database and applications could reside on a separate secure server. The user could be handed over from the web server to the database/application server when requesting information, accessing personal information or ordering online. To ensure the highest level of transaction security, all communication could be through a Virtual Private Network (VPN) connection. Furthermore, the website could be secured with an SDS ID token based authentication system.

The website could consist of an HTML user interface with the site constructed with the Coldfusion (CFML) programming environment. The use of the Coldfusion programming environment would allow for access to the database, scalability and the use of COBRA with the applications server. The applications server could contain the data interpretation and related programs as well as a Cybercash application (a commerce transaction program). An Oracle database could be partitioned on the applications server.

### Diabetes counselling

A counselling centre (30) may provide constant diabetes advice for the user of the wireless diabetes phone 24 hours a day, 7 days a week, independent of the location of the patient. The counselling centre (30) comprises a network of centres staffed with diabetes educators who are trained and competent in interpreting diabetes data and advising patients how to deal with their disease and insulin dosage. If a user desires assistance with a diabetes-related matter, he may access the diabetes counselling centre through the wireless diabetes phone (20). The user could send glucose and other diabetes information via the wireless diabetes phone (20) to the counselling centre (30), where a trained counsellor could obtain the most recent glucose results from the glucose meter through the phone and, if available, insulin and dietary intake together with a question from the user. The counsellor could calculate an appropriate treatment response and assists the user to interpret his data and make the correct decisions regarding his treatment, i. e. insulin dosage or glucose intake. The patient may select between two assistance modes provided by the counselling centre (30). The user may then be prompted to send the data.

The first mode, automated data interpretation, comprises a text message that is sent to the wireless diabetes phone (20) of the patient. When using the first mode of the counselling service, the glucose meter must be in communication with the phone so that the phone can send an e-mail to the data base which in turn identifies the correct data-retrieval software package for the meter of the user. This software package may be sent to the user's phone and used to access the data from the glucose meter. These data may then be sent to the database, and an updated record of the user's glucose results may be sent to a counsellor, along with a question selected by the phone user. The data could then be analysed by automated data interpretation utilising proprietary software and assistance could be posted on the user's website or sent as an e-mail message to the user's phone.

According to the second mode, voice counselling, the counsellor reviews the data, then calls the patient and provides interactive verbal assistance. Automated data interpretation utilising proprietary software could be used as a screen to determine when a patient needs immediate counsellor intervention. In the case of an obvious emergency, the counsellor may decide to call the 911 dispatch centre.

As an alternative to sending the data to the counselling centre, the patient may send the data to a health care professional in order to allow his/her physician to stay in full control of his/her therapy.

### Diabetes Supervision

Consulting the database allows a caretaker or health care professional to view the glucose results and verify the frequency of testing of the patient. Consultation may occur on demand of the caretaker (40) or health care professional, who could use a computer to view the patient's records stored in the database (11). For example, this feature may be useful for a parent interested in monitoring the management activity of a child at summer camp.

According to one embodiment, the health care professional or managed care provider can supervise the testing patterns and test results of patients and reward patients for frequent testing and/or maintenance of blood glucose levels within a prescribed range for a predetermined period of time.

### Diabetes News

According to another aspect, the diabetes management system of the present invention also provides a diabetes news service to users through the wireless diabetes phone. This service provides the latest diabetes news to keep patients informed about developments and risks associated with the disease.

### On-phone Ordering of Supplies

According to another aspect of the invention, the wireless diabetes phone (20) may be utilised for convenient ordering of diabetic products and third party billing for the diabetic products. During a sign-up process for the wireless diabetes phone (20), a patient can enter the products he or she most often purchases for his or her diabetes. The wireless diabetes phone (20) may include an "order on-phone" menu on the display screen, which lists these products. As can be seen in figure 2, upon selecting the items he or she wants to purchase from the phone screen, a wireless order may be sent to the central monitoring centre (10), such as MobileDiabetes™, which then forwards the order as a secured data transfer to the retail chain (180) or supplier identified by the user for fulfilment. The retailer (180) may send back confirmation of the order reception to the database, which may send a confirmation e-mail back to the wireless diabetes phone (20) of the user. The billing and shipping information may be stored in the database for the monitoring centre (10), which would facilitate the ordering process. In addition, the database may contain patient and insurance information, which would allow automatic and direct third party billing for the on-phone order.

### Retailers

The wireless diabetes phone (20) and management system of the present invention provides unique advertising opportunities to retailers (60), as the messages sent over the wireless diabetes phone (20) may be targeted in nature. For example, commercial messages (190) from medical device suppliers can be sent out to the users of the phone, who comprise a very specific group of active measuring diabetics. The central database (11) records information regarding the store and/or chain in which the glucose meter (20a) was purchased and can target store and chain specific messages to these diabetics. The usage and demographic data compiled by the database can be of great value to a glucose meter company or other diabetic supplier to direct their marketing and sales campaigns toward a particular population. Targeted advertising, specific to diabetics of a certain age and sex, shopping in a certain chain, testing at least a certain number of times a day, can be of great value to all suppliers in the diabetes market. For example, the message: "This week store brand syringes are 30% off" can be easily sent to all store or chain customers having the wireless diabetes phone (20) of the present invention. The targeted advertising provides direct, customised ad-messages, which reach customers in real time. Messages can be tailored to a user's profile information and may be provided to the user through e-mail or incorporated into any message sent to the user from the database. For example, a message could be attached to a message sent by the counselling centre or the order on-phone service. A message confirming that the advertising message has been opened or read could then be sent back to the monitoring centre (10). As an example of the service, the targeted advertising may be utilised to reward users of a particular brand with a discount or to promote a new product of the brand most used by the user of the wireless diabetes phone. Alternatively, the targeted advertising messages may be sent to users of a competitor's product to convert the users to a particular brand of products.

### Health Programs

In addition to serving as a data gathering system from the glucose meter (20a) to the database (11), the wireless diabetes phone (20) may serve as a communication, motivation and reminder tool for the diabetes patient. The wireless diabetes phone (20) may be utilised to provide patient coaching and motivational or compliance programs (200) encouraging patients to closely manage their diabetes. The monitoring centre (10) provides the health improvement programs (200) based on personal coaching to improve the diabetes management of the diabetic patients using the wireless diabetes phone (20). For example, the health program may provide dietary or exercise-related advice, information, reminders and motivation to the user. The health programs may be e-mail based programs where automatic replies from the database are sent to the user to provide prompt feedback that the data have been received by the database. In order to upgrade the programming of the phone to a more advanced data-gathering system, software could be downloaded from the website.

Among other features provided by the wireless diabetes phone (20) and glucose meter (20a) can be a reminder function to alert the patient or a caretaker that a measurement or an administration of medication is needed. The wireless diabetes phone (20) may also provide other diabetes-related messages and reminders. For example, if the database information or the insurer reveals that a year has passed since the patient's last eye examination, the wireless diabetes phone (20) may be utilised to send reminders to the diabetic patient to have an eye examination, an issue that is particularly important for diabetics. The phone may also be used to provide a message to the user to encourage them to check the web-site for updates or personalised messages.

The wireless diabetes phone and/or glucose meter stores measurements taken from the diabetic patient until the measurements are transmitted to a remote computer, cleared by a user or cleared remotely by an operator of the automated monitoring centre (10). The wireless diabetes phone can transmit information to the remote computer at a predetermined time or when polled by the patient or other user of the diabetes management system. The automated monitoring centre (10) can initiate contact with the patient by either e-mail or voice communication at a predetermined time or when a measurement is outside a predetermined range, indicating a potential problem. The automated monitoring centre (10) directly polls the wireless diabetes phone (20) for measurements at regular intervals or when data are not received from the patient before a predetermined time. According to one practice of the invention, the automated monitoring centre (10) initiates an emergency procedure when a measurement is not timely received and the patient cannot be contacted. The central database (11) could also be used to report a patient's progress to their insurer.

The diabetes management system and diabetes wireless phone of the present invention provide significant benefits and advantages to diabetic individuals. The present invention provides a diabetic patient with constant access to professional counselling and assistance whenever it is needed, that is when a problem is imminent. The assistance can be provided independent of the location of the patient at an economically justifiable cost. Through the use of the wireless diabetes phone, a diabetic patient can quickly, automatically and accurately transfer data from a glucose meter to a professional counsellor. The phone increases the mobility, self-efficacy and independence of the patient while helping the patient to improve control over diabetic symptoms. The present invention promotes an improved health status and quality of life by reducing short-term complications (e.g. hypo- and hyper- glycaemia) and long-term complications associated with diabetes. The present invention further facilitates continuity of care through education and empowerment, reduces anxiety related to diabetic symptoms, resolves uncertainty about insulin dosage decisions and promotes an independent lifestyle. Through implementation of the system of the present invention, diabetes-related hospitalisations, emergency room visits and associated costs for both short and long term complications are reduced. Furthermore, the present invention facilitates the overall management of the disease, including access to supplies.

In addition, in the insulin market there is a definite resistance to changing insulin regimens, brands and products. One method to facilitate the change over for both the physician and the diabetic is to provide counselling and monitor the patient through the transition phase. The present invention provides an excellent solution for insulin manufacturers to support the launch of their new insulin products.

The technology of the present invention can also be applied in the field of pregnancies, where early risk identification combined with targeted patient education and support has proven to reduce the incidence of prenatal and maternal complications (for example, premature delivery, low birth weight and caesarean section) and the overall cost of maternal care. The present invention could be used to support physician and health plan goals for maternity care by providing comprehensive telephone-based maternal education, monitoring and counselling throughout pregnancy and after delivery. Use of the present invention could help to reduce the incidence of premature delivery and caesarean sections, reduce the number of low birth weight babies and neonatal intensive care admissions and improve physician and health care member satisfaction.

These examples are meant to be illustrative and not limiting. The present invention has been described by way of example, and modifications and variations of the exemplary embodiments will suggest themselves to skilled artisans in this field without departing from the scope of the invention. Features and characteristics of the above-described embodiments may be used in combination. The preferred embodiments are merely illustrative and should not be considered restrictive in any way. The scope of the invention is to be measured by the appended claims, rather than the preceding description, and all variations and equivalents that fall within the range of the claims are intended to be embraced therein.

## Claims

1. A system for monitoring and assisting in the treatment of a patient, independent of the location of the patient, comprising:
a monitoring centre;
a meter for measuring the level of an analyte in said patient; and
a communications device adapted for communication with said monitoring centre and said meter, wherein said communications device is adapted to transmit information related to said analyte level in said patient to said monitoring centre,
wherein said communications device and said monitoring centre are connected through a Virtual Private Network (VPN) connection.

2. The system of claim 1, wherein the monitoring centre comprises a website secured with an SDS ID token based authentication system.

3. The system of claim 1, wherein said patient is a diabetic patient, said analyte is glucose and said meter is a glucose meter.

4. The system of any preceding claim, wherein said communications device is a wireless communications device.

5. The system of any of claims 1 to 3, wherein said communications device transmits information via the internet.

6. The system of any preceding claim, wherein said monitoring centre stores said information related to an analyte level in a database.

7. The system of any preceding claim, wherein said monitoring centre stores patient-related information regarding the patient in the database.

8. The system of claim 7, wherein said monitoring centre stores diabetes-related information regarding a diabetic patient in the database.

9. The system of any preceding claim, further comprising a caretaker communications device, allowing a caretaker of said patient to access the database.

10. The system of claim 9, wherein the monitoring centre notifies said caretaker in case of an emergency by means of the caretaker communications device.

11. The system of any preceding claim, further comprising:
a counselling centre for providing assistance to said patient by means of said communications device.

12. The system of claim 11, wherein said counselling centre notifies emergency services if a dangerous analyte level is read by the meter.

13. The system of any preceding claim, wherein said communications device is adapted to transmit a health program to said patient from said monitoring centre.

14. The system of any preceding claim, further comprising a retail store, wherein said monitoring centre is adapted to transmit advertising messages from said retail store to said patient.

15. The system of any preceding claim, further comprising a retail store, wherein said monitoring centre is adapted to transmit information relating to products from said retail store to said patient.

16. The system of any preceding claim, wherein said communications device automatically contacts emergency services if the information related to an analyte level reveals a dangerous condition in the patient.

17. A device capable of receiving and transmitting an analyte level measurement of a patient, comprising:
a communications device configured to receive the analyte level of the patient from a meter and transmit the analyte level of the patient to a remote monitoring centre via a Virtual Private Network (VPN) connection.

18. The device of claim 17, wherein the patient is diabetic, the analyte is glucose and the meter is a glucose meter.

19. The device of claims 17 or claim 18 wherein the communications device is a wireless communications device.

20. The device of any of claims 17 to 19, wherein the communications device includes an alarm for notifying an emergency service if said analyte level is outside of a predetermined range.

21. The device of any of claims 17 to 20, wherein the communications device includes storage for storing analyte level measurements of the patient.
